# EUROPEAN PATENT SPECIFICATION

(11) **EP 2 997 926 B1**
(45) Date of publication and mention of the grant of the patent: **04.09.2019**
(21) Application number: 13863530.5
(22) Date of filing: 12.12.2013
(51) Int. Cl.: A61B 17/17, A61G 13/12, A61B 34/20, A61B 17/16

(54) **GUIDING AND HOLDING DEVICE FOR MINIMUM INCISION FOOT SURGERY**
FÜHRUNGS- UND HALTEVORRICHTUNG FÜR FUSSCHIRURGIE MIT MINIMALER INZISION
DISPOSITIF DE GUIDAGE ET DE FIXATION DESTINÉ À LA CHIRURGIE MINI-INVASIVE DU PIED

(30) Priority: 13.12.2012 ES 201201245 P
(43) Date of publication of application: 23.03.2016
(73) Proprietor: INSPOMED, S.L., 46008 Valencia (ES)
(72) Inventor: Ferrer Torregrosa, Javier, 46018 Valencia (ES); García Vicente, Sergio, 46183 La Eliana - Valencia (ES)
(74) Representative: Alier Benages, Elisabet
(86) International application number: PCT/ES2013/070877
(87) International publication number: WO 2014/091054

(56) References cited:
- WO-A1-2011/029934
- WO-A1-2011/029934
- WO-A1-2012/146814
- US-A- 5 813 977
- US-A- 5 881 730
- US-A1- 2002 049 443
- US-A1- 2007 118 116
- US-A1- 2007 118 116
- US-B1- 6 589 242

## Description

### Object of the invention

The object of the present invention is to provide surgeons specialize in podiatry and foot trauma, with a guiding and holding device to facilitate them the correct execution of minimal incision surgical operations, ensuring that execution and the postoperative period are developed according to planning.

### Technical field

The present invention refers to a device for percutaneous minimal incision foot surgery

### Background of the invention

MIS (Minimal Incision Surgery) or percutaneous surgery is a surgical method that allows surgical operations through minimal incisions between 4 and 5 mm, reducing the trauma caused to the nearby tissues. These incisions can be cured without suture, though, to ensure a proper scar, a suture of 1 to 3 simple points is usually done.

This technique is used for percutaneous surgery of soft tissue (tenotomy, tendon lengthening, tendon debridement or capsulotomy) or bone parts (exostosectomies or osteotomy).

The practice of these percutaneous surgery techniques should be carried out by podiatrists or surgeons with experience who know exactly the anatomical structures involved in each surgery, with extensive knowledge of biomechanics to foresee how the biomechanical function will look like after surgery. The surgeon/podiatrist has to do with great precision surgical gestures, because they can injure healthy parts or even carry out mutilations. In the same way the choice of complete or incomplete osteotomy is marked by the experience and the skill of the professional. This will plan, for example, an incomplete osteotomy, and it will only look like in this way if he/she is dexterous enough to not break it. Each digital deformity is different; the number and spatial orientation of the necessary osteotomy for correction are determined by the position of each phalanx, accommodation resets that tendon and capsular anatomical elements suffer, and the degree of articulate involvement. Osteotomies are performed through corrective wedges, the opening of which is located at the opposite side to the deformity leaving intact the portion of cortical contrary to the same, but weakened enough to allow its closure, providing a proper contact of the resulting fragments and avoiding their displacement, in this way, we will achieve the necessary correction by plantarflexing, dorsiflexing, adducing or abducing the bone segment to be treated.

To make these minimal incision surgery techniques, the following process is followed: Anamnesis;
Physical examination;
Complementary examinations;
Planning: By using the previous information it is defined how to solve the pathology, the surgery will be performed, which follow these steps (incision site, angle of approach, approach path, percutaneous surgical gestures);
Anesthesia;
Positioning of the patient;
Surgery of soft or bony parts;
Radiological control;
Suture, disinfection and fixation;
Review.

The instruments used for minimal incision foot surgery is: Anesthetic injection;
Scalpel and scalpel handle;
Files (Lewis, Bell, Polokoff, MiniPolokoff);
Burrs (Shannon-Isham, short Shannon 44, medium Shannon 44, long Shannon 44, Brophy, Wedge 3.1 and 4.1);
Elevators (Freer, Curvo, Locke, Sebileau);
Haemostatic forceps;
Needle holder, curved, straight needles and thread;
Scissors;
Dissection forceps;
Motorized instruments: Micro-motor and handpiece with reducing adapter; Radiological instruments (fluoroscope);
And the required surgical material.

In the execution of minimal incision surgeries, it is common to not achieve precisely the correction of the deformity, since it requires a very high learning curve. Some of the errors that occur in such interventions do not have solution. The reason why most of these errors are committed is an improper execution of the surgical operation by the surgeon. The execution of the MIS surgery has great difficulty since the surgeon does not display what he/she does, guided only with the radiological control and the patient's external anatomy.

Errors that occur during the surgical operation in foot interventions in MIS surgery: Damage to blood vessels, nerves or tendons, since these do not appear in the radiological control, the surgeon is based on his experience.

The standardization of techniques, regardless of the degree of severity the angle of the osteotomy should be enlarged, the number of failures will be increased. Excessive surgical aggressiveness on soft tissue and bony parts will cause postoperative inflammatory processes with longer duration.

Any osteotomy badly designed or poorly executed, could produce a retardation of ossification or no connection.

If the execution and consolidation of the osteotomy is not exact, it will cause a shortening of the bone segment treated, increasing for example the percentage of pressure transfers to the foot.

When planning an incomplete osteotomy, this can be broken and moved causing misalignment and closure of subsequent osteotomies.

Break of proximal osteotomies causing an overcorrection or misalignment.

In the minimal incision foot surgeries, errors that do not comply with the premises of the Wolfs Law for pressure for optimum bone consolidation are committed, both in the execution of the osteotomy and in the postoperative bandage, and the bone can finish in an improper position.

Multiple patents that define devices intended for guiding the surgical instruments in a wide variety of surgical operations such as hip, knee, skull, etc., have been found in the State of the art. Several patents that have relevance in terms of surgical guiding are indicated:
The patent "Drill guide for angled trajectories" N° WO2010/062634, proposes a device to guide the bit drilling the bone in the hole of a bone junction plate. This guiding device is not intended for minimal incision foot surgery.

The patent "Surgical jig" N° WO2005/041785, proposes a guiding device to perform an osteotomy on the first metatarsal of the foot. Surgery performed with this device is not of minimal incision, a cut between 4 and 10 cm must be made and scrape the surface of the bone to fix the device; all of this linked to the problems which may be generated by open surgery, in which multiple tissues and blood vessels are cut. There are MIS techniques to perform equivalent diaphyseal osteotomies. A minimal incision osteotomy can not be carried out with this device
The patent "Surgical instrument for facilitating accurate osteotomy cuts in bone and method for utilizing same" N° US005112334 , proposes a guiding device for carrying out an osteotomy at a certain angle. To fix this device to bone, a 2-4 cm incision must be performed and scrap the surface of the bone. A minimal incision osteotomy can not be carried out with this device.

WO2011/029934 discloses a guiding and fastening device for minimal incision surgery of the foot having at least one guide piece which limits the operating zone of the conventional surgical instrument. The guide piece has at least one punctual guiding through hole in the plane of X-Y axes, the through hole extending in the direction of Z axis. The device comprises a cover that is fastened and attached to the foot on which surgery is performed. The cover is formed by at least two parts which secure the foot, the parts being provided with conventional mutual attachment and detachment means. The cover has at least one through hole in which the guide piece is fitted in the correct position and orientation The cover and the guide piece comprise conventional union means that allows them to assembled/ disassembled. However, the device does not disclose drainage channels.

The closet prior art, US 2007/0118116, discloses a guiding and holding device with at least one guide part which limits the region of operation of conventional surgical instruments ; at least one through hole of the guide part; an upper wall with an angle "a" that limits the inclination in which the surgical instruments can be positioned; an stopper that limits the depth to which the surgical instrument can be introduced; at least one lower wall which is oval-shaped or at angle "b"; an envelope formed by two parts that grab the foot; at least one through hole of the envelope, in which the guide part is fitted in the proper position and orientation and several bores which are suitable as drainage channels or absorption areas. However, the bores are not connected.

### Description of the invention

The present invention provides a guiding and holding device as defined by claim 1 intended for different surgery operations of minimal incision foot surgery, which solves several problems that occur during the surgical operation and the postoperative period.

The device described in the present invention reduces the chance for error since it is performed a minimal incision surgery, as planned previously to the surgical operation, reducing the error factors depending on the ability and skill of the surgeon. This device helps to reduce the use of radiological control, minimizing the radiation received by the patient, the medical team involved in the surgery.

Fixing of guiding devices in the bone structures are inconsistent with MIS operations, so the device described in the present invention performs the guiding in minimal incision surgeries MIS, without fixing the guiding part in contact with a bone structure. This device is used in feet, since these extremities have very few soft areas surrounding the bones, for this reason, the envelope is adjusted and immobilizes the area that covers the foot.

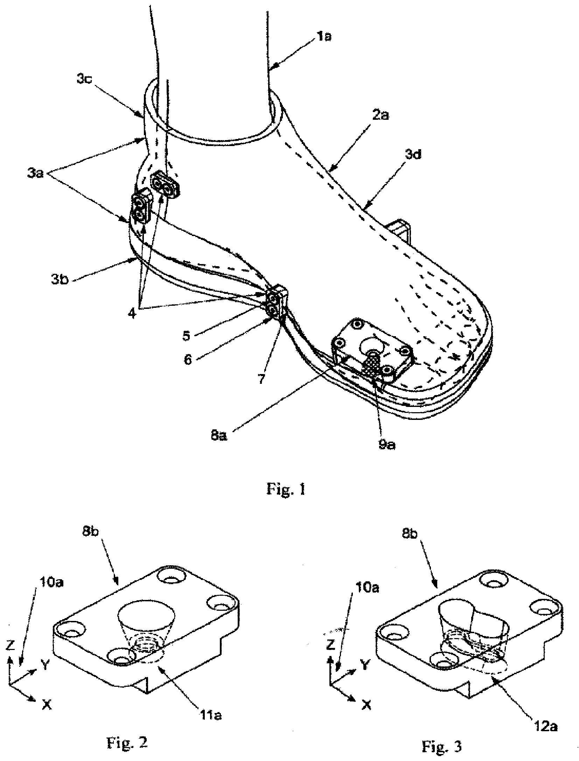

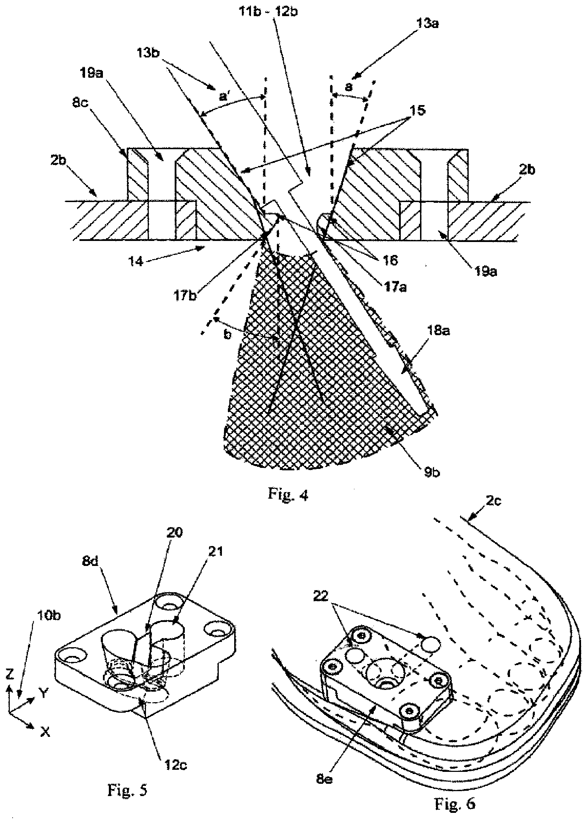

The device described in the present invention for guiding and holding in minimal incision foot surgery (1a) (FIG. 1) has at least one guide part (8) (8a,FIG. 1), which limits the region of operation (9a) (FIG. 1) of the conventional surgical instruments such as scalpes, rasp and motorized burrs, that are used for minimal incision foot surgeries. This guide part avoids that errors dependent on the skill of the surgeon are committed, achieving perfect angles in osteotomies. The use of several guide parts during surgical operation is very useful, since different parts of the operation will be carried out safely. The guide part (8) (8b FIG. 2) has at least one through hole (11a) (FIG. 2) for point guiding or that forms a route (12a) (FIG. 3) in the plane of XY axes (10a) (FIG. 2 and 3), this hole is though-type in the direction of the Z axis (10a) (FIG. 2 and 3). The through hole (11b)-(12b) (FIG. 4) of the guide part (8) (8c in FIG. 4), has at least one upper wall (15) (FIG. 4) with an angle "a" (13a) (FIG. 4) that limits the inclination in which the surgical instruments can be positioned (18a) (FIG. 4), this angle "a" may vary along the walls in different values of angle "a' " (13b) (FIG. 4). The upper wall (15) ( FIG. 4) is straight, concave, convex or varies along the wall, depending on the region of operation (9b) (FIG. 4) of the surgical instruments. The through hole (11b)- (12b) (FIG. 4), has a stopper (16) (FIG. 4) that limits the depth to which the surgical instruments can be introduced. The through hole (11b)- (12b) (FIG. 4), has at least one lower wall which is oval-shaped (17a) (FIG. 4) or at angle "b" (17b) (FIG. 4), and which does not produce a limitation to the surgical instruments greater than the upper walls (15) (FIG. 4). The surface of the stopper (16) (FIG. 4) may be straight, concave, or convex, or vary along the stopper.

The device of the present invention has an envelope (2) (FIG. 1) held and attached to the foot, in which the surgery will be performed, providing a constant relationship between the area where the surgery will be performed and the guide part. This envelope ensures that the position and orientation of the guide part is always correct. The envelope is formed by at least two parts (3a) (FIG. 1) which grab the foot. The parts have conventional means for being joined with and separated (4) (FIG. 1) from each other, such as screws, clearance setting, quick connectors, closing mechanism, strips, tabs, bolts, magnets, velcro, zipper or clamps. The envelope (2) (2b in FIG. 4) has at least one through hole (14) (FIG. 4), in which the guide part (8) (8c in FIG. 4) is fitted in the proper position and orientation. The envelope and guide part have conventional joining means (19a) ( FIG. 4) that allow its assembly and/or disassembly such as screws, clearance setting, quick connectors, electromechanical connectors, closing mechanism, bolts or strips.

The guide part, the envelope and the connecting elements are preferably of transparent or translucent material to the visible electromagnetic radiation and/or conventional radiation used for radiological tests.

To be able to verify the correct placement of guide part or the envelope with respect to the foot, radiology tests are used. The guide part or the envelope may, in its interior or the surface, include at least one insert made of material radio-opaque to the radiation of the radiological tests, with preferential sphere, cylinder or dome shapes.

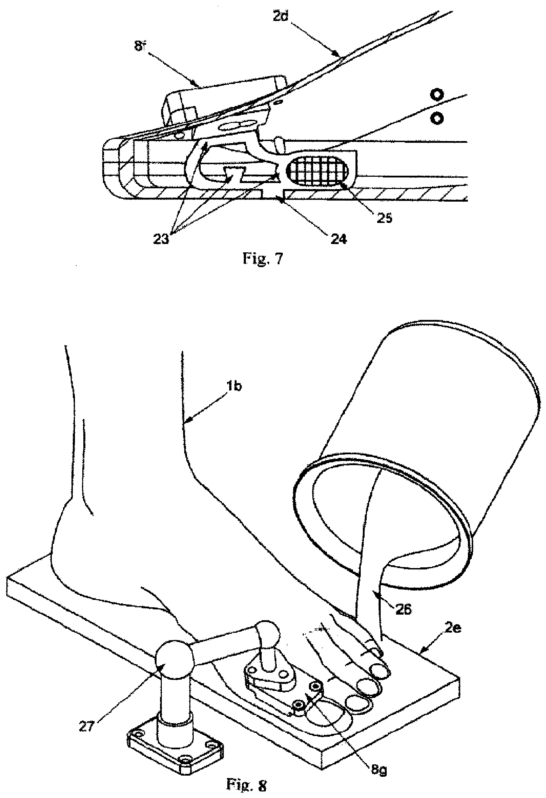

During surgery, excess blood or fluid can hinder or prevent execution of the surgical operation using the device of the present invention, therefore in certain surgeries, the drainage of blood or body fluids must be ensured. This problem is solved by having at least a fluid drainage. The guide part (8) (8f in FIG. 7) or envelope (2) (2d in FIG. 7) have at least one drainage channel (23) (FIG. 7) in the area in contact with the skin and connected to at least one through hole on the outside (24) (FIG. 7) of the envelope. The guiding and holding device further comprises at least one absorption area in at least one of the drainage channels, and standard absorbent material or culture media placed in the absorption area to analyze the sterilization of the surgical operation. The conventional absorbent material may comprise gauze, tissues, compresses, salts or cotton.

The first version of envelope (2) (2e in FIG. 8) is of a conventional material (26) (FIG. 8) such as alginate, plaster, epoxy or polymer, which is applied in a liquid state and solidifies in less than 30 minutes, adapting to the exact shape of the foot. The guide parts (8) (8g in FIG. 8) are placed in the correct position and orientation, with conventional means as an articulated multi-axis arm (27) (FIG. 8), before the envelope material is solidified. By applying again more material (26) (FIG. 8) after its solidification, more than one piece for the envelope is obtained that can be assembled and disassembled. This way of obtaining the envelope, is a common technique for the realization of complex molds or for the reproduction of parts. This invention makes a very different use of this technique, since the mold is the envelope to be obtained. The materials are analogous to those used for obtaining molds of teeth, which do not create allergic problems or irritations.

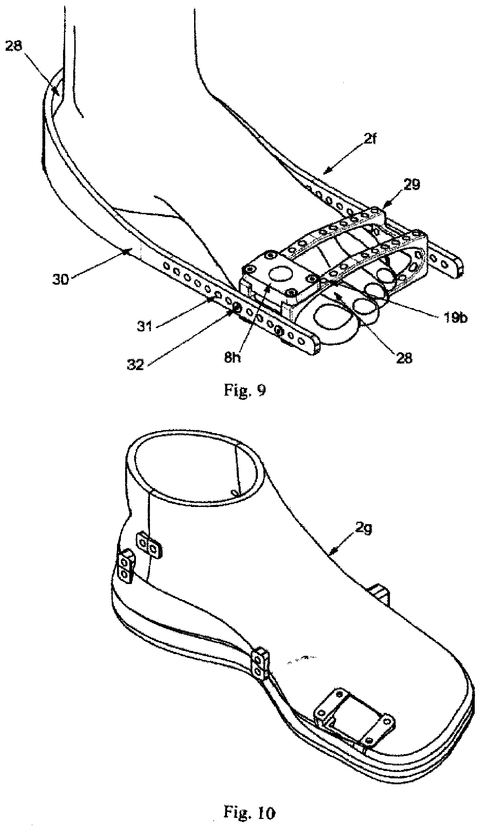

The second version of envelope can rely on or complement the first version. This version resolves the envelope (2) (2f in FIG. 9) with an exoskeleton or standardized structure for different feet sizes, with possibility of adjustment. It has at least a clamping part (30) (

FIG. 9) which has at least one holding area (28) (FIG. 9) which is coupled to the foot, limiting its mobility with respect to the guide part (8) (8h in FIG. 9). The clamping part joins and regulates in a position to at least one fixation for guide parts (29) (FIG. 9), with at least one conventional union (32) (FIG. 9) and adjustment (31) (FIG. 9) as rail with union by screws, telescopic tubes with union by grippers. The fixation for guide parts (29) (FIG. 9) has at least one union (19b) (FIG. 9) where the guide part (8h) (FIG. 9) is coupled in a predetermined position and orientation. This is a version that aims to reduce costs involved in performing a specific envelope for each patient. This envelope will have limitations for certain surgical operations.

The third version of envelope can rely on or complement the first and/or second version. First, three-dimensional information of foot structure is collected, these data allow obtaining a perfectly adjusted envelope in which easily include accessories and manufacture it with CAM techniques, rapid 3D prototyping or any technological advance that achieves the expected result. Information of structure and three-dimensional foot composition is obtained with conventional techniques such as CAT (computerized axial tomography), CT (computerized tomography), MRI (magnetic resonance imaging), ultrasound, x-ray or 3D scanner. The surgery is planned with a computerized system, the guide part is defined, its position-orientation and auxiliary elements. This version is resolved with an exoskeleton or custom structure (2) (2g in FIG. 10) for the foot and the surgical techniques of minimal incision to be carried out. With the data of the three-dimensional structure, three-dimensional internal composition of the foot and surgery planning, a custom envelope is constructed with conventional CAM techniques (computer-aided manufacturing) such as 3D printing, machining, laser stereolithography or laser sintering.

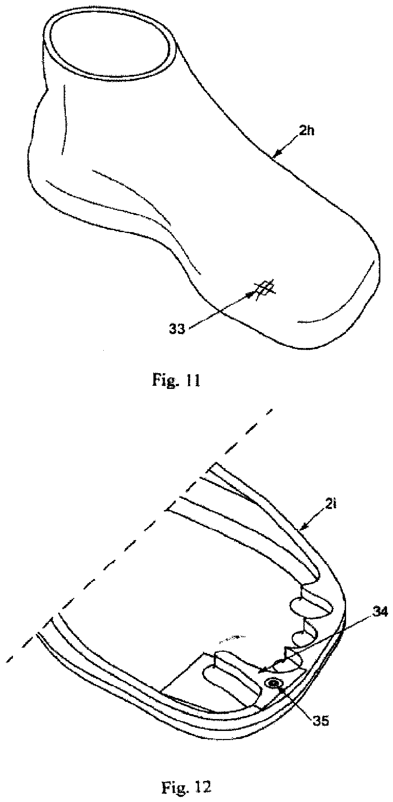

The fourth version of envelope can rely on or complement the first and/or second and/or third version. This version resolves the envelope (2) (2h in FIG. 11) with a semi-rigid sock or glove, having on its surface at least to mark (33) (FIG. 11) such as ink, projections and recesses, indicating where the guide parts are placed. New rapid prototyping systems allow the use of semi-rigid materials and at the same time are able to include dyes during the development of the prototype.

Accessories or auxiliary elements are envisioned for the envelope. In order to change the position in which the area to be treated in the surgery is located. There are occasions where it is necessary to separate fingers, others in which part of the operation is performed with the finger stretched and then folded, others in which a first action from the top and the second from a side, others in which, during the same surgery, more than one surgical gesture is carried out, even multi-techniques are used to correct different pathologies, being necessary to move a finger. The use of an auxiliary element that can be fixed and removed easily from the envelope facilitates the surgical operation. This situation is resolved with at least one auxiliary coupling element (34) (FIG. 12) by which fingers are placed into the suitable position for surgery. These auxiliary coupling elements will comprise a specific form according to the needs of operation, such as wedges, sheets or gauze. The auxiliary element is joined to the envelope with conventional joining means (35) (FIG. 12) that allow its assembly and/or disassembly such as screws, clearance setting, quick connectors, electromechanical connectors, closing mechanism, bolts, strips or velcro.

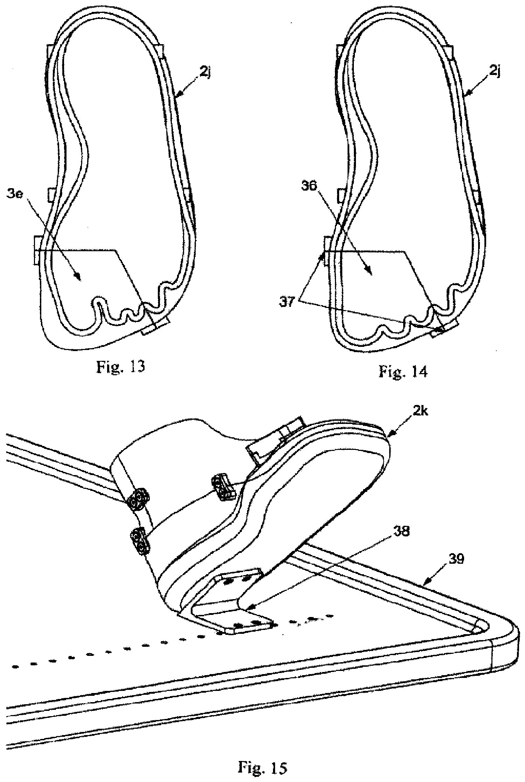

Accessories or auxiliary elements are envisioned for the envelope. To solve the problems of poor placement of the bones in the postoperative period and causing a bad union or the absence of the union. Surgical intervention is performed with a part in the envelope (3e) (FIG. 13), which after the surgical operation is replaced by an auxiliary element with the appropriate geometry to achieve a correct position of soldering of bones. This problem is resolved having in the envelope (2) (2j in FIG. 14) at least of an auxiliary coupling element (36) (FIG. 14) by which fingers are placed in the suitable position after surgery. The auxiliary coupling element is joined to the envelope with conventional means (37) (FIG. 14) that allow its assembly and/or disassembly such as screws, clearance setting, quick connectors, electromechanical connectors, closing mechanism, bolts, strips. In certain circumstances, all parts of the envelope can be replaced obtaining an envelope to a conventional postoperative bandage, but with the advantage of being detachable and able to perform cures and verify the evolution of surgery.

Parts to fix the foot through the envelope and prevent the patient from moving during surgery are envisioned. This fixation is solved having at least one part (38) (FIG. 15) to fix and place the envelope (2) (2k in FIG. 15) in a determined position and orientation with regard to the surgery table-chair (39) (FIG. 15). The part (38) (FIG. 15) is an articulated arm, wedge or bracket. The fixation and placement part (38) (FIG. 15) is attached to the envelope (2) (2k in FIG. 15) and the surgery table-chair (39) (FIG. 15), with conventional means such as screws, clearance setting, quick connectors, closing mechanism, strips, tabs, bolts, magnets, velcro, vacuum, zipper or clamps.

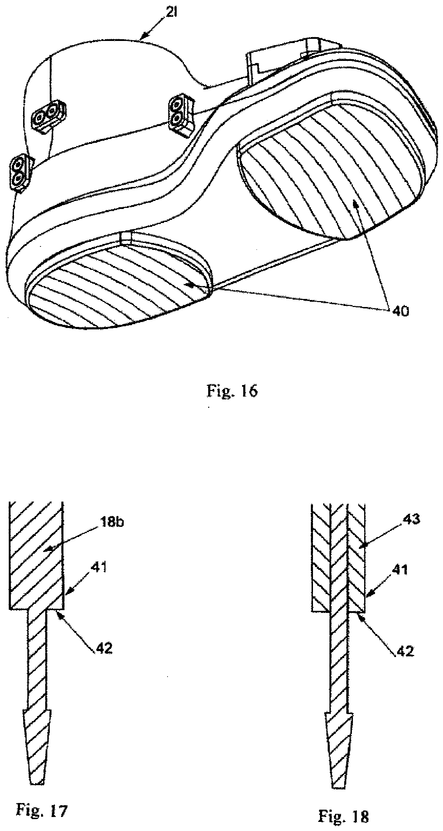

It is envisioned that the envelope or part of this can be used in the postoperative period and during the postoperative bone soldering/union, incorporating a sole or support area to the envelope so that the patient can support the envelope without the need of additional elements,. The support of the foot is solved having at least one sole (40) (FIG. 16), attached to the envelope (2) ((21 in FIG. 16) by conventional means such as screws, clearance setting, quick connectors, closing mechanism, strips, tabs, bolts, magnets, velcro, zipper or clamps.

Along with this device described in the present invention, surgical instruments adapted for better interaction with the guide part can be used. The surgical instruments used with the guide part (8) (8c in FIG. 4) has a wall (41) (FIG. 17 and 18) and a stopper (42) (FIG. 17 and 18) that limits the movement of the surgical instruments with the upper wall (15) (FIG. 4) and the stopper (16) (FIG. 4) of the guide part (8) (8c in FIG. 4). The wall (41) (FIG. 17 and 18) and the stopper (42) (FIG. 17 and 18) are part of the instruments (18b) (FIG. 17), as a complement (43) (FIG. 18) that adapts and fixes to existing instruments.

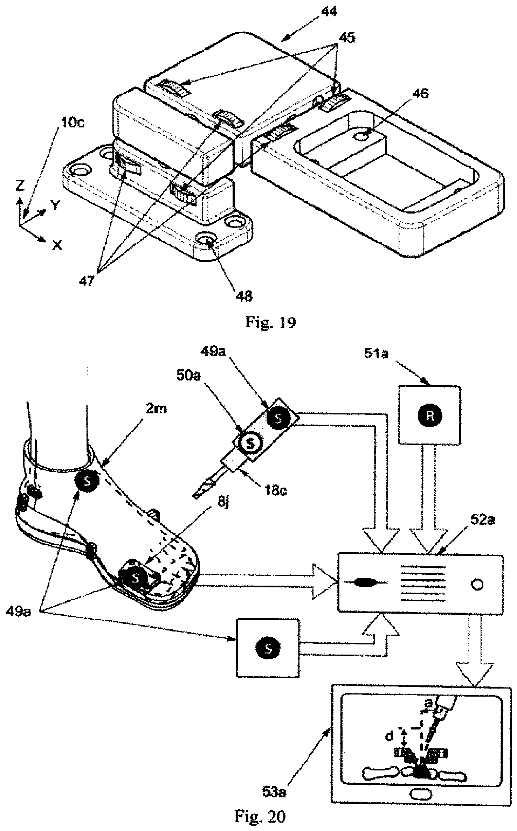

During the surgery, small misalignments may arise in the positioning and orientation of guide part with respect to the area where the surgery is going to be performed. To solve these possible deviations between the envelope and guide part, by which to be able to correct these deviations, is envisioned. The present invention has a positioning and rotation interface (44) (FIG. 19) between the guide part and envelope, by which the adjustments of position and rotation of guide part with respect to the envelope are carried out. The positioning interface (44) (FIG. 19) has one union (48) (FIG. 19) to the envelope and other union (46) (FIG. 19) to the guide part with the same conventional means between the enveloping union and the guide part. The positioning interface has conventional means to move (45) (FIG. 19) in at least one de los X, Y, Z axes (10c) ( FIG. 19), such as worn screws, pulleys, gears, linear motors or hydraulic pistons. The positioning interface (44) (FIG. 19) has conventional means to rotate (47) (FIG. 19) in at least one of the X, Y, Z axes (10c) (FIG. 19) such as screws, steering wheels with gears, worm screws, pulleys, gears, linear motors or rotary engines.

In order to facilitate the proper execution of the surgical operation and provide useful information to the surgeon, a version in which the device of the present invention has sensors, emitters, markers and receptors that collect information in real time is envisioned. The data collected are processed by electronic equipment and communicated to the surgeon by a screen. With this help the surgeon can know the position, orientation, speed, pressure, speed of the drill, among others, according to the sensors used. The device of the present invention has at least one sensor-emitter-marker (49a) (FIG. 20) in a known position and at least one receptor (51a) (FIG. 20). With the sensor-emitter-marker and receptor, the position and orientation of the guide part (8) (8j in FIG. 20), of the surgical instruments or the positioning interface are determined with regard to the foot envelope (2) (2m in FIG. 20). The sensor-emitter-marker and the receiver are conventional such as: compass IC sensors, gyroscope IC sensors, laser emitter-receptor triangulation, laser emitter-receptor laser scanning, optical receptor structured light scanning, ultrasonic emitter-receptor triangulation, position-orientation markers opaque to x-rays, fluoroscopy or position-orientation markers for artificial vision receptor. Information from the sensors-emitters-markers and/or receptor, is transferred to a specific electronic control equipment (52a) (FIG. 20) or conventional computer (52a) (FIG. 20 ), where data are processed, obtaining the display on at least one screen (53a) (FIG. 20), of data from the position and orientation of the foot, the guide part, the surgical instruments, the positioning interface and the envelope. By using surgical instruments with sensors (50a) (FIG. 20), additional technical data of the surgical operation are obtained. With conventional sensors such as tachometers, accelerometers, pressure sensors, strain gauge, triaxial strength sensor, temperature sensors, encoder, proximity sensors, inertial sensors or electromagnetic radiation sensors.

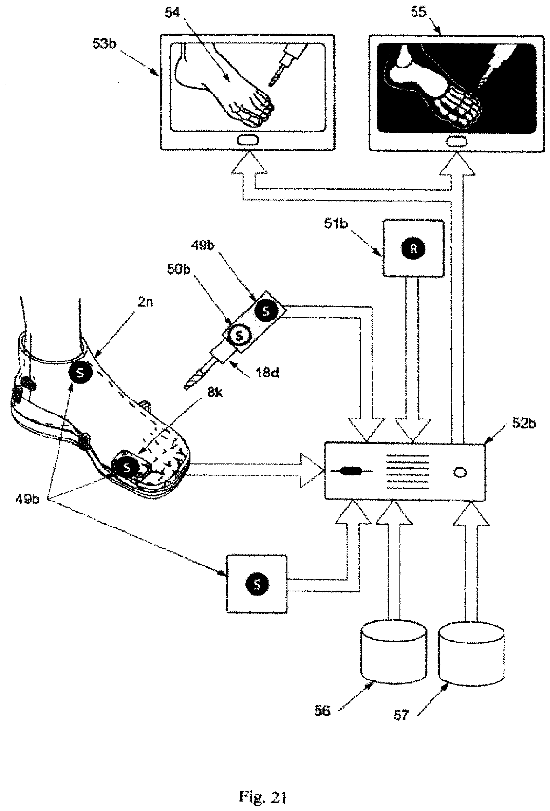

A surgery with greater guarantees of success is possible if the surgeon sees the execution of the surgery. With virtual reality or augmented reality techniques the surgeon can see a real-time simulation of the surgical operation. This possibility is a great step forward for MIS foot surgery, which is practically blind. The present invention has the virtual visualization in real time during the execution of the foot surgery with conventional techniques of virtual or augmented reality (54) (FIG. 2). This visualization is performed with a specific electronic control equipment (52b) (FIG. 21) or conventional computer (52b) (FIG. 20), that receives the data of the three-dimensional structure of the foot (56) (FIG. 21) obtained from conventional radiological tests such as CAT (Computerized Axial Tomography), CT (Computerized Tomography) or MRI (Magnetic Resonance Imaging). The computer (52b) (FIG. 21) processes the information received from the sensors-emitter-marker (49b) (FIG. 21) and receptors (51b) (FIG. 21), the instrument sensors (50b) (FIG. 21), the data from radiological tests (56) (FIG. 21) and surgery planning data (57) (FIG. 21). With this data, a virtual representation is generated on at least one screen (53b) (FIG. 21) what is happening inside the foot during the surgical operation. Through the use of conventional radiological tests such as fluoroscopy during surgical operation, the real execution of the surgery is verified on at least one screen (55) (FIG. 21).

To simplify the tasks of planning and execution of the MIS surgery in feet, a specific software for this purpose is used. This software or computer program has the following general functions:
Planning the surgery, in a three-dimensional environment, calculating, obtaining and viewing a simulation of the result of surgery.

Collecting patient information and the data from radiological or biometric tests. Determining the surgical instruments to be used.

For the use of envelopes or custom guide parts, generates data for its CAM machining, rapid 3D prototyping or any technological advance that achieves the expected result. Guides the surgeon with virtual or augmented reality techniques during the surgical operation.

### Brief description of the drawings:

For the better understanding of what is described in this specification, drawings are attached in which just by way of example, a relationship of figures of the guiding and holding device for minimal incision foot surgery is depicted.

### Description of figures

FIG. 1 : Guiding and holding device for minimal incision surgery, placed in the patient's foot.
FIG. 2 : Guide part with point hole.
FIG. 3 : Guide part with hole following a path.
FIG. 4 : Detail of section of the guide part and the envelope, where it can be seen how the movement, orientation and depth of surgical instruments is limited to a region of operation.
FIG. 5 : Guide part with additional hole to introduce surgical instruments.
FIG. 6 : Envelope and guide part with holes for endoscopy.
FIG. 7 : Section of the envelope and guide part where drainage channels are seen.
FIG. 8 : Envelope performed as a mold with material that solidifies with the exact shape of the foot, to which it can be attached the guide part in its suitable position with respect to the foot and different accessories.
FIG. 9 : Envelope with exoskeleton or standard structure that couples and holds to the foot, the guide part can be attached in multiple predefined positions and different accessories.
FIG. 10 : Envelope obtained with biometric data of the foot and performed with CAM techniques. The accessories and coupling points for the guide part can be obtained in this envelope.
FIG. 11 : Semi-rigid envelope as a sock or glove, with marks to connect the guide part and different accessories.
FIG. 12 : Auxiliary coupling element, fixed to the envelope; in this example it is a separator for the thumb with respect to the rest of the fingers.
FIG. 13 : Envelope with a coupling element for surgery, in this example toes are placed in a certain position to perform the surgical operation.
FIG. 14 : Envelope with a coupling element after surgery, in this example the toes are placed in a certain position so that the soldering of the bones is in the defined position and orientation.
FIG. 15 : Fixing part that keeps fixed the envelope in the surgical table-chair in a defined position and orientation.
FIG. 16 : Sole for foot envelopes, with which the patient can walk during and after surgery.
FIG. 17 : Surgical instruments with stoppers that are part of the same, for use with the guide part.
FIG. 18 : Surgical instruments with stoppers that are added, for use with the guide part.
FIG. 19 : Positioning and orientation interface of the guide part with respect to the envelope.
FIG. 20 : Envelope, guide part and surgical instruments with sensor-emitter-marker and receptor, to obtain technical data which are processed and displayed on a monitor to the surgeon during surgery.
FIG. 21: Envelope, guide part and surgical instruments with sensor-emitter-marker and receptor, to obtain technical data that are processed together with the patient's planning and biometric data, to obtain simulation on a monitor of the surgery execution. Visualization of the actual execution with radiological techniques.

### Description of a preferred embodiment:

A preferred embodiment is cited by way of example being independent of the materials used, the methods employed in the manufacture and accessories which may have the guiding and holding device for minimal incision food surgery; provided that they do not affect its essentiality.

It is stated a preferred embodiment:
It is shown as an example, a patient suffering from Hallux valgus in the right foot, the surgeon, after carrying out the appropriate tests, decides that adequate treatment is a distal osteotomy of the first metatarsal of the big toe, in which he will have to make a 15 degrees correction.

After a magnetic resonance, three-dimensional data of the structure of the right foot are obtained; with this information and the use of a specific software, the surgeon plans the surgery. The surgeon determines the region of operation in the bone, the point of incision and surgical instruments to remove a wedge of 15 degrees. The software simulates the result of surgery and resulting soldering of the bone after surgery. The surgeon can determine to include different accessories or features to the envelope and the guide part. With the information from the surgical operation and accessories, the software determines the exact specifications that are necessary in the envelope and the guide part.

It is determined that an envelope of 6 mm thick, made up of 5 pieces, is required: Sole part envelope (3b) (FIG. 1) that covers the entire sole of the foot for surgery. This envelope has two drainage channels communicating with a through hole to the outside so that the fluids flow out.

Heel part envelope (3c) (FIG. 1) that covers the top of the heel. Instep part envelope (3d) (FIG. 1) that covers the area of the instep of the foot.

This part of the envelope has a through hole (14) (FIG. 4), in which the guide part (8) (8c in FIG. 4) is fitted. It also has four threaded connection points (19a) (FIG. 4) to fix the guide part. This piece is for surgery.

Post-surgery sole part envelope that covers the entire sole of the foot, for the postoperative period, where the thumb is placed for a correct bone soldering. Post-surgery instep part envelope that covers the instep of the foot, for the postoperative period, where the thumb is placed for a correct bone soldering.

All parts of the envelope have an adding (7) (FIG. 1) with threaded holes, in order to attach them with plates of two holes (6) (FIG. 1) and screws (5) (FIG. 1). These plates and screws are in nylon, which is transparent to x-rays,

The guide part limits the region of operation to that defined by the surgeon. Two guide parts that will fit in the same envelope are carried out, the first for the cut with a scalpel and the second for milling machine:
The guide part for the cut with a scalpel has a through hole in the form of route (12b) (FIG. 4), with a straight upper wall (15) (FIG. 4) at a angle of 45 degree, with a stopper (16) (FIG. 4) which limits the depth of the scalpel to the bone, with a lower wall (17b) ( FIG. 4) at an angle of 60 degrees and the straight surface of the stopper. It also has 4 holes (19a) (FIG. 4), to attach with screws to the instep part envelope (3d) (FIG. 1).

The guide part for the osteotomy with the milling machine has a through hole in the form of route (12b) (FIG. 4), with a straight upper wall (15) (FIG. 4) at a angle of 7.5 degree in each wall, with a stopper (16) (FIG. 4) which limits the depth of the end of the bone up to 2 mm, with a lower wall (17b) (FIG. 4) at an angle of 60 degrees and the concave surface of the stopper that reproduces the shape of the bone. It also has 4 holes (19a) (FIG. 4) to attach with screws to the instep part envelope (3d) (FIG. 1).

The manufacture of parts of the envelope and the guide part is done with equipment for rapid 3D prototyping, by laser sintering. The resulting polymer of this manufacturing process is transparent to x-rays. During the construction of the envelope and the guide part, QR codes (49b) (FIG. 21) are included in order to apply augmented reality techniques.

The surgical instruments, the scalpel and the milling machine have QR codes (49b) (FIG. 21) for augmented reality. The QR codes are a way of encoding information for sensors of augmented reality; codes can be used with another type of encoding which indicate that code is that displayed.

For the realization of the surgical operation planned, in addition to the usual medical instruments for these interventions, there are 3 video cameras (51b) (FIG. 21) connected to a computer (52b) (FIG. 21) with a specific software, which interprets the data from the planning, the structure of the foot obtained with magnetic resonance imaging and video cameras, getting the display (54) (FIG. 21) of the virtual simulation of surgical operation, that allows the surgeon to view what is happening on the inside of the patient's foot on a screen (53b) (FIG. 21).

## Claims

1. Guiding and holding device for minimal incision foot surgery having :
- at least one guide part (8) which, in use, limits the region of operation (9a) of conventional surgical instruments; the guide part (8) has at least one through hole (11a) for point guiding that forms a route (12a) in the plane of XY axes (10a), this hole is through-type in the direction of the Z axis (10a); the through hole (11b)-(12b) of the guide part (8) has at least one upper wall (15) with an angle "a" (13a) that limits the inclination in which the surgical instruments can be positioned (18a), this angle "a" may vary along the walls in different values of angle "a"' (13b); the upper wall (15) is straight, concave, convex or varies along the wall, depending on the region of operation (9b) of the surgical instruments; the through hole (11b)-(12b) has a stopper (16) that limits the depth to which the surgical instruments can be introduced; the through hole (11b)-(12b) has at least one lower wall which is oval-shaped (17a) or at angle "b" (17b), and which does not produce a limitation to the surgical instruments greater than the upper walls (15), and
- an envelope (2) which, in use, is held and fixed to the foot in which the surgery will be performed; the envelope is formed by at least two parts (3a) that grab the foot, the parts have conventional means for being joined with and separated (4) from each other; the envelope (2) has at least one through hole (14), in which the guide part (8) is fitted in the proper position and orientation; the envelope and guide part have conventional joining means (19a) that allow its assembly and/or disassembly; wherein the guide part (8) or the envelope (2) have at least one drainage channel (23) in the area in contact with the skin and connected to at least one through hole on the outside (24) of the envelope;
**characterized in that** the guiding and holding device further comprises at least one absorption area (25) in at least one of the drainage channels, and standard absorbent material or culture media placed in the absorption area to analyze the sterilization of the surgical operation.

2. Guiding and holding device according to the definition of claim 1, **characterized in that** the guide part (8d) has at least one through hole of surgical instruments (21), in the direction of the Z axis (10b), greater than the part of the surgical instruments that performs the surgery. The guiding through hole (12c) and through hole of instrument (21), are attached or connected by a path (20) allowing passing instruments from a hole to the other.

3. Guiding surgical device according to the definition of claim 1, **characterized in that** the guide part (8e) or the envelope (2c) has at least one through hole (22) to introduce the equipment into the correct position and orientation. The through holes (22) must have a diameter larger than the part of the equipment/surgical instruments that is introduced.

4. Guiding and holding device according to the definition of claim 1, **characterized in that** the envelope (2e) is made of a conventional material (26) that applies on the foot (1b) and solidifies adapting to the exact shape of the foot. The guide parts (8g) are placed in the correct position and orientation, with conventional means, before the envelope material is solidified. By applying again more material (26) after its solidification, more than one piece for the envelope is obtained that can be assembled and disassembled.

5. Guiding and holding device according to the definition of claim 1, **characterized in that** the envelope (2f) is an exoskeleton or standardized structure for different foot sizes, with possibility of adjustment, Has at least one clamping part (30) which has at least one clamping area (28) that couples to the foot limiting its mobility with respect to the guide part (8h). The clamping part joins and regulates in a position to at least one fastening for guide parts (29), with at least one conventional union (32) and adjustment (31). The fastening for guide parts (29), has at least one union (19b) where the guide part (8h) is coupled in a predetermined position and orientation.

6. Guiding and holding device according to the definition of claim 1, **characterized in that** the envelope (2h) is a semi-rigid sock or glove, having on its surface at least a mark (33) indicating where the guide parts are placed. Partial complements of adjustable envelopes (2e), standard exoskeletons (2f) or custom exoskeletons (2g) are added to the sock- or glove-type envelope (2h).

7. Guiding and holding device according to the definition of any of preceding claims, **characterized in that** the envelope (2i) has at least one auxiliary coupling element (34) with a specific shape according to the needs of the operation. The auxiliary element is joined to the envelope with conventional joining means (35) that allow its assembly and/or disassembly.

8. Guiding and holding device according to the definition of any of preceding claims, **characterized in that** the envelope (2j) has at least one auxiliary coupling element (36) by which the fingers are placed in the correct position after surgery. The auxiliary coupling element is joined to the envelope with conventional means (37) that allow its assembly and/or disassembly.

9. Guiding and holding device according to the definition of any of preceding claims, **characterized in that** it has at least one part (38) for fixing and placing the envelope (2k) in a determined position and orientation with regard to the surgery table-chair (39); the part (38) is an articulated arm, wedge or bracket. The fixation and placement part (38) is attached to the envelope (2k) and the surgery table-chair (39), with conventional means.

10. Guiding and holding device according to the definition of any of preceding claims, **characterized in that** it has at least one sole (40), attached to the envelope (21) by conventional means.

11. Guiding and holding device according to the definition of any of preceding claims, **characterized in that** it has a positioning and rotation interface (44) between the guide part and the envelope, by which the adjustments of position and rotation of guide part with respect to the envelope are carried out. The positioning interface (44) has a union (48) to the envelope and other union (46) to the guide part with the same conventional means between the enveloping union and the guide part. The positioning interface has conventional means to move (45) in at least one of the axis X, Y and Z (10c). The positioning interface (44) has conventional means to rotate (47) in at least one of the axis X, Y and Z (10c).

12. Guiding and holding device according to the definition of any of preceding claims, **characterized in that** it has at least one sensor-emitter-marker (49a) in a known position and at least one receptor (51a). With the sensor-emitter-marker and receptor, the position and orientation of the guide part (8j), the surgical instruments or the positioning interface is determined with regard to the foot envelope (2m). The sensor-emitter-marker and the receiver are conventional; the information from the sensors-emitters-markers and/or receptor, is transferred to a specific electronic control equipment (52a) or conventional computer (52a), where data are processed, obtaining the display on at least one screen (53a) of data from the position and orientation of the foot, the guide part, the surgical instruments, the positioning interface and the envelope. The surgical instruments through the incorporation of conventional sensors (50a), obtains additional technical data of the surgical operation.

13. Guiding and holding device according to the definition of claim 12, **characterized by** having virtual visualization in real time during the execution of the foot surgery with conventional techniques of virtual or augmented reality (54). This visualization is performed with a specific electronic control equipment (52b) or conventional computer (52b), that receives the data of the three-dimensional structure of the foot (56), obtained from conventional radiological tests. The computer (52b) processes the information received from the sensors-emitter-marker (49b) and receptors (51b), the instrument sensors (50b), the data from radiological tests (56) and surgery planning data (57). With these data, a virtual representation what is happening inside the foot during the surgical operation is generated on at least one screen (53b).

## Patentansprüche

1. Führungs- und Haltevorrichtung für fusschirurgie mit minimaler inzision:
- mindestens einen Führungsteil (8), der in Verwendung den Wirkbereich (9a) von herkömmlichen chirurgischen Instrumenten einschränkt; wobei der Führungsteil (8) mindestens ein Durchgangsloch (11a) zur Punktführung aufweist, das eine Route (12a) in der Ebene von XY-Achsen (10a) bildet, dieses Loch in der Richtung der Z-Achse (10a) durchgehend ist; das Durchgangsloch (11b)-(12b) des Führungsteils (8) mindestens eine obere Wand (15) mit einem Winkel "a" (13a) aufweist, der die Neigung begrenzt, in welcher die chirurgischen Instrumente positioniert werden können (18a), dieser Winkel "a" entlang der Wände in verschiedenen Werten des Winkels "a" (13b) variieren kann; die obere Wand (15) gerade, konkav, konvex ist oder entlang der Wand in Abhängigkeit vom Wirkbereich (9a) der chirurgischen Instrumente variiert; das Durchgangsloch (11b)-(12b) einen Anschlag (16) aufweist, der die Tiefe begrenzt, bis zu welcher die chirurgischen Instrumente eingeführt werden können; das Durchgangsloch (11b)-(12b) mindestens eine untere Wand aufweist, die oval geformt (17a) oder in einem Winkel "b" (17b) ist, und die keine größere Einschränkung der chirurgischen Instrumente als die oberen Wände (15) bewirkt, und
- eine Hülle (2), die in Verwendung am Fuß, an welchem die Operation durchgeführt wird, gehalten und fixiert wird; wobei die Hülle durch mindestens zwei Teile (3a) gebildet wird, die den Fuß ergreifen, die Teile herkömmliche Mittel aufweisen, um miteinander verbunden und voneinander getrennt zu werden (4); die Hülle (2) mindestens ein Durchgangsloch (14) aufweist, in welches der Führungsteil (8) in der geeigneten Position und Ausrichtung eingepasst wird; die Hülle und der Führungsteil herkömmliche Verbindungsmittel (19a) aufweisen, die ihre Montage und/oder Demontage ermöglichen;
**wobei** der Führungsteil (8) oder die Hülle (2) mindestens einen Ableitkanal (23) im Bereich in Kontakt mit der Haut und verbunden mit mindestens einem Durchgangsloch auf der Außenseite (24) der Hülle aufweist;
**dadurch gekennzeichnet, dass** die Führungs- und Haltevorrichtung ferner mindestens einen Absorptionsbereich (25) in mindestens einem der Ableitkanäle und standardmäßiges Absorptionsmaterial oder Kulturmedium, das im Absorptionsbereich angeordnet ist, zum Analysieren der Sterilisation des chirurgischen Eingriffs umfasst.

2. Führungs- und Haltevorrichtung nach Anspruch 1, **dadurch gekennzeichnet, dass** der Führungsteil (8d) mindestens ein Durchgangsloch für chirurgische Instrumente (21) in der Richtung der Z-Achse (10b) aufweist, das größer als der Teil der chirurgischen Instrumente ist, der die Operation durchführt. Das Führungsdurchgangsloch (12c) und das Durchgangsloch des Instruments (21) werden durch einen Weg (20), der den Durchlass von Instrumenten von einem Loch zum anderen ermöglicht, angebracht oder verbunden.

3. Führungs- und Haltevorrichtung nach Anspruch 1, **dadurch gekennzeichnet, dass** der Führungsteil (8e) oder die Hülle (2c) mindestens ein Durchgangsloch (22) zum Einführen der Geräte in die korrekte Position und Ausrichtung umfasst. Die Durchgangslöcher (22) müssen einen Durchmesser aufweisen, der größer als der Teil der Geräte/chirurgischen Instrumente ist, der eingeführt wird.

4. Führungs- und Haltevorrichtung nach Anspruch 1, **dadurch gekennzeichnet, dass** die Hülle (2e) aus einem herkömmlichen Material (26) hergestellt ist, das auf den Fuß (1b) aufgebracht wird und sich unter Anpassung an die exakte Form des Fußes verfestigt. Die Führungsteile (8g) werden in der korrekten Position und Ausrichtung mit herkömmlichen Mitteln angeordnet, bevor sich das Hüllenmaterial verfestigt hat. Durch erneutes Aufbringen von mehr Material (26) nach seiner Verfestigung wird mehr als ein Stück für die Hülle erhalten, das montiert und demontiert werden kann.

5. Führungs- und Haltevorrichtung nach Anspruch 1, **dadurch gekennzeichnet, dass** die Hülle (21) ein Exoskelett oder eine standardisierte Struktur für verschiedene Fußgrößen mit der Möglichkeit zur Anpassung ist. Sie weist einen Klemmteil (30) auf, der mindestens einen Klemmbereich (28) aufweist, der mit dem Fuß gekoppelt wird und seine Mobilität in Bezug auf den Führungsteil (8h) einschränkt. Der Klemmteil verbindet und reguliert sich in einer Position zu mindestens einer Befestigung für Führungsteile (29) mit mindestens einer herkömmlichen Verbindung (32) und Anpassung (31). Die Befestigung für Führungsteile (29) weist mindestens eine Verbindung (19b) auf, woran der Führungsteil (8h) in einer vorgegebenen Position und Ausrichtung gekoppelt wird.

6. Führungs- und Haltevorrichtung nach Anspruch 1, **dadurch gekennzeichnet, dass** die Hülle (2h) ein halbsteifer Strumpf oder Handschuh ist, der auf seiner Oberfläche mindestens eine Markierung (33) aufweist, die angibt, wo die Führungsteile angeordnet werden. Teilkomplemente von anpassbaren Hüllen (2e), standardmäßigen Exoskeletten (2f) oder maßgefertigten Exoskeletten (2g) werden zur Hülle (2h) vom Strumpf- oder Handschuhtyp hinzugefügt.

7. Führungs- und Haltevorrichtung nach einem der vorhergehenden Ansprüche, **dadurch gekennzeichnet, dass** die Hülle (2i) mindestens ein Hilfskoppelelement (34) mit einer spezifischen Form gemäß den Erfordernissen des Eingriffs aufweist. Das Hilfskoppelelement wird mit der Hülle mit herkömmlichen Verbindungsmitteln (35) verbunden, die seine Montage und/oder Demontage ermöglichen.

8. Führungs- und Haltevorrichtung nach einem der vorhergehenden Ansprüche, **dadurch gekennzeichnet, dass** die Hülle (2j) mindestens ein Hilfskoppelelement (36) aufweist, durch welches die Finger nach der Operation in der korrekten Position angeordnet werden. Das Hilfselement wird mit der Hülle mit herkömmlichen Mitteln (37) verbunden, die seine Montage und/oder Demontage ermöglichen.

9. Führungs- und Haltevorrichtung nach einem der vorhergehenden Ansprüche, **dadurch gekennzeichnet, dass** sie mindestens einen Teil (38) zum Fixieren und Anordnen der Hülle (2k) in einer bestimmten Position und Ausrichtung in Bezug auf den Operationstisch/-stuhl (39) aufweist; wobei der Teil (38) ein gelenkiger Arm, Keil oder Bügel ist. Der Fixier- und Anordnungsteil (38) wird an der Hülle (2k) und dem Operationstisch/-stuhl (39) mit herkömmlichen Mitteln angebracht.

10. Führungs- und Haltevorrichtung nach einem der vorhergehenden Ansprüche, **dadurch gekennzeichnet, dass** sie mindestens eine Sohle (40) aufweist, die durch herkömmliche Mittel an der Hülle (21) angebracht ist.

11. Führungs- und Haltevorrichtung nach einem der vorhergehenden Ansprüche, **dadurch gekennzeichnet, dass** sie eine Positionier- und Drehschnittstelle (44) zwischen dem Führungsteil und der Hülle aufweist, durch welche die Anpassungen von Position und Drehung des Führungsteils in Bezug auf die Hülle ausgeführt werden. Die Positionierschnittstelle /44) weist eine Verbindung (48) mit der Hülle und eine andere Verbindung (46) mit dem Führungsteil mit den gleichen herkömmlichen Verbindungsmitteln zwischen der Hüllverbindung und dem Führungsteil auf. Die Positionierschnittstelle weist herkömmliche Mittel zur Bewegung (45) in mindestens einer der Achsen X, Y und Z (10c) auf. Die Positionierschnittstelle (44) weist herkömmliche Mittel zur Drehung (47) in mindestens einer der Achsen X, Y und Z (10c) auf.

12. Führungs- und Haltevorrichtung nach einem der vorhergehenden Ansprüche, **dadurch gekennzeichnet, dass** sie mindestens einen Sensor-Emitter-Marker (49a) in einer bekannten Position und mindestens einen Rezeptor (51a) aufweist. Mit dem Sensor-Emitter-Marker und dem Rezeptor werden die Position und die Ausrichtung des Führungsteils (8j), der chirurgischen Instrumente oder der Positionierschnittstelle in Bezug auf die Fußhülle (2m) bestimmt. Der Sensor-Emitter-Marker und der Rezeptor sind herkömmlich; die Informationen von den Sensoren-Emittern-Markern und/oder dem Rezeptor werden an ein spezifisches elektronisches Steuergerät (52a) oder einen herkömmlichen Computer (52a) übertragen, wo Daten verarbeitet werden, um die Anzeige auf mindestens einem Bildschirm (53a) von Daten von der Position und Ausrichtung des Fußes, des Führungsteils, der chirurgischen Instrumente, der Positionierschnittstelle und der Hülle zu erhalten. Die chirurgischen Instrumente erhalten durch die Einbindung herkömmlicher Sensoren (50a) zusätzliche technische Daten des chirurgischen Eingriffs.

13. Führungs- und Haltevorrichtung nach Anspruch 12, **gekennzeichnet durch** Aufweisen von virtueller Visualisierung in Echtzeit mit herkömmlichen Techniken virtueller oder erweiterter Realität (54) während der Ausführung der Fußoperation. Diese Visualisierung wird mit einem spezifischen elektronischen Steuergerät (52b) oder einen herkömmlichen Computer (52b) durchgeführt, das/der die Daten der dreidimensionalen Struktur des Fußes (56) empfängt, die aus herkömmlichen radiologischen Tests erhalten werden. Der Computer (52b) verarbeitet die Informationen, die von den Sensoren-Emittern-Markern (49b) und den Rezeptoren (51b), den Instrumentensensoren (50b), den Daten aus radiologischen Tests (56) und den chirurgischen Planungsdaten (57) empfangen werden. Mit diesen Daten wird auf dem mindestens einen Bildschirm (53b) eine virtuelle Darstellung dessen erzeugt, was sich während des chirurgischen Eingriffs innerhalb des Fußes ereignet.

## Revendications

1. Dispositif de guidage et de fixation destiné à la chirurgie mini-invasive du pied ayant :
- au moins une pièce de guidage (8) qui, lorsqu'elle est utilisée, limite la zone d'opération (9a) des instruments chirurgicaux conventionnels ; la pièce de guidage (8) a au moins un trou traversant (11a) pour le guidage ponctuel qui forme un parcours (12a) dans le plan des axes XY (10a), ce trou est du type traversant dans le sens de l'axe Z (10a) ; le trou traversant (11b)-(12b) de la pièce de guidage (8) a au moins une paroi supérieure (15) avec un angle « a » (13a) qui limite l'inclinaison dans laquelle les instruments chirurgicaux peuvent être positionnés (18a), cet angle « a » peut varier le long des parois dans différentes valeurs de l'angle « a » (13b) ; la paroi supérieure (15) est droite, concave, convexe ou varie le long de la paroi, en fonction de la zone d'opération (9b) des instruments chirurgicaux ; le trou traversant (11b) - (12b) a une butée (16) qui limite la profondeur à laquelle les instruments chirurgicaux peuvent être introduits ; le trou traversant (11b) - (12b) a au moins une paroi inférieure qui est de forme ovale (17a) ou à l'angle « b » (17b) et qui n'entraîne pas de limitation aux instruments chirurgicaux supérieure aux parois supérieures (15) et
- une enveloppe (2) qui, lorsqu'elle est utilisée, est maintenue et fixée au pied dans laquelle la chirurgie sera effectuée ; l'enveloppe est constituée d'au moins deux pièces (3a) qui saisissent le pied, les pièces ont des moyens conventionnels pour être reliées et séparées (4) les unes des autres ; l'enveloppe (2) a au moins un trou traversant (14), dans lequel la pièce de guidage (8) est introduite dans la bonne position et la bonne orientation ; l'enveloppe et la pièce de guidage ont des moyens de liaison conventionnels (19a) qui permettent leur assemblage et/ou désassemblage ;
**dans lequel** la pièce de guidage (8) ou l'enveloppe (2) ont au moins un canal de drainage (23) dans la zone en contact avec la peau et relié à au moins un trou traversant à l'extérieur (24) de l'enveloppe ;
**caractérisé en ce que** le dispositif de guidage et de maintien comprend en outre au moins une zone d'absorption (25) dans au moins l'un des canaux de drainage, et un matériau absorbant standard ou un milieu de culture placé dans la zone d'absorption pour analyser la stérilisation de l'opération chirurgicale.

2. Dispositif de guidage et de fixation selon la définition de la revendication 1, **caractérisé en ce que** la pièce de guidage (8d) a au moins un trou traversant d'instruments chirurgicaux (21), dans la direction de l'axe Z (10b), plus grand que la pièce des instruments chirurgicaux qui effectue la chirurgie. Le trou traversant de guidage (12c) et le trou traversant de l'instrument (21), sont joints ou raccordés par une voie (20) permettant le passage d'instruments d'un trou à l'autre.

3. Dispositif chirurgical de guidage selon la définition de la revendication 1, **caractérisé en ce que** la pièce de guidage (8e) ou l'enveloppe (2c) a au moins un trou traversant (22) pour introduire l'équipement dans la bonne position et la bonne orientation. Les trous traversants (22) doivent avoir un diamètre plus large que la partie de l'équipement/des instruments chirurgicaux qui est introduite.

4. Dispositif de guidage et de fixation selon la définition de la revendication 1, **caractérisé en ce que** l'enveloppe (2e) est réalisée à partir d'un matériau conventionnel (26) qui est appliqué sur le pied (1 b) et qui se solidifie en s'adaptant à la forme exacte du pied. Les pièces de guidage (8g) sont placées dans la bonne position et la bonne orientation, avec des moyens conventionnels, avant que le matériau de l'enveloppe ne se solidifie. En appliquant à nouveau plus de matériau (26) après sa solidification, plus d'une pièce pour l'enveloppe est obtenue qui peut être assemblée et désassemblée.

5. Dispositif de guidage et de fixation selon la définition de la revendication 1, **caractérisé en ce que** l'enveloppe (21) est un exosquelette ou une structure standardisée pour différentes tailles de pied, avec la possibilité de réglage. A au moins une pièce de serrage (30) qui a au moins une zone de serrage (28) qui est couplée au pied limitant sa mobilité par rapport à la pièce de guidage (8h). La pièce de serrage se relie et se règle dans une position à au moins un élément de fixation pour les pièces de guidage (29), avec au moins une union conventionnelle (32) et de réglage (31). La fixation pour les pièces de guidage (29), a au moins une union (19b) où la pièce de guidage (8h) est couplée dans une position et une orientation prédéterminées.

6. Dispositif de guidage et de fixation selon la définition de la revendication 1, **caractérisé en ce que** l'enveloppe (2h) est une chaussette ou un gant semi-rigide, ayant à sa surface au moins une marque (33) indiquant où les pièces de guidage sont placées. Des compléments partiels d'enveloppes ajustables (2e), des exosquelettes standard (2f) ou des exosquelettes personnalisés (2g) sont ajoutés à l'enveloppe de type chaussette ou gant (2h).

7. Dispositif de guidage et de fixation selon la définition selon l'une quelconque des revendications précédentes, **caractérisé en ce que** l'enveloppe (2i) a au moins un élément de couplage auxiliaire (34) avec une forme spécifique selon les besoins de l'opération. L'élément auxiliaire est relié à l'enveloppe avec des moyens d'union conventionnels (35) qui permettent son assemblage et/ou désassemblage.

8. Dispositif de guidage et de fixation selon la définition selon l'une quelconque des revendications précédentes, **caractérisé en ce que** l'enveloppe (2j) a au moins un élément de couplage auxiliaire (36) par lequel les doigts sont placés dans la bonne position après la chirurgie. L'élément de couplage auxiliaire est relié à l'enveloppe avec des moyens conventionnels (37) qui permettent son assemblage et/ou désassemblage.

9. Dispositif de guidage et de fixation selon la définition selon l'une quelconque des revendications précédentes, **caractérisé en ce qu'**il a au moins une pièce (38) pour la fixation et la mise en place de l'enveloppe (2k) dans une position et une orientation déterminées par rapport à la table-fauteuil de chirurgie (39) ; la pièce (38) est un bras articulé, un coin ou support. La pièce de fixation et de mise en place (38) est jointe à l'enveloppe (2k) et à la table-fauteuil de chirurgie (39), avec des moyens conventionnels.

10. Dispositif de guidage et de fixation selon la définition selon l'une quelconque des revendications précédentes, **caractérisé en ce qu'**il a au moins une semelle (40), jointe à l'enveloppe (21) par des moyens conventionnels.

11. Dispositif de guidage et de fixation selon la définition selon l'une quelconque des revendications précédentes, **caractérisé en ce qu'**il a une interface de positionnement et de rotation (44) entre la pièce de guidage et l'enveloppe, par lequel les réglages de position et de rotation de la pièce de guidage par rapport à l'enveloppe sont réalisés. L'interface de positionnement (44) a une union (48) avec l'enveloppe et une autre union (46) avec la pièce de guidage, avec les mêmes moyens conventionnels entre l'union enveloppante et la pièce de guidage. L'interface de positionnement a des moyens conventionnels pour se déplacer (45) dans au moins l'un des axes X, Y et Z (10c). L'interface de positionnement (44) a des moyens conventionnels pour tourner (47) dans au moins l'un des axes X, Y et Z (10c).

12. Dispositif de guidage et de fixation selon la définition selon l'une quelconque des revendications précédentes, **caractérisé en ce qu'**il a au moins un capteur-émetteur-marqueur (49a) dans une position connue et au moins un récepteur (51a). Avec le capteur-émetteur-marqueur et le récepteur, la position et l'orientation de la pièce de guidage (8j), des instruments chirurgicaux ou de l'interface de positionnement sont déterminées par rapport à l'enveloppe du pied (2m). Le capteur-émetteur-marqueur et le récepteur sont conventionnels ; les informations provenant des capteurs-émetteurs-marqueurs et/ou du récepteur, sont transférées vers un équipement de commande électronique spécifique (52a) ou un ordinateur conventionnel (52a), où les données sont traitées, obtenant l'affichage sur au moins un écran (53a) de données provenant de la position et l'orientation du pied, la pièce de guidage, les instruments chirurgicaux, l'interface de positionnement et l'enveloppe. Les instruments chirurgicaux par l'incorporation de capteurs conventionnels (50a), obtiennent des données techniques supplémentaires de l'opération chirurgicale.

13. Dispositif de guidage et de fixation selon la définition de la revendication 12, **caractérisé par** le fait d'avoir une visualisation virtuelle en temps réel pendant l'exécution de la chirurgie du pied avec des techniques conventionnelles de réalité virtuelle ou augmentée (54). Cette visualisation est réalisée avec un équipement de commande électronique spécifique (52b) ou un ordinateur classique (52b), qui reçoit les données de la structure tridimensionnelle du pied (56), obtenues à partir de tests radiologiques conventionnels. L'ordinateur (52b) traite les informations reçues des capteurs-émetteurs-marqueurs (49b) et des récepteurs (51b), des capteurs d'instrument (50b), des données de tests radiologiques (56) et des données de planification de la chirurgie (57). Avec ces données, une représentation virtuelle de ce qui se passe à l'intérieur du pied pendant l'opération chirurgicale est générée sur au moins un écran (53b).
